(19) Europäisches Patentamt
European Patent Office
Office européen des brevets

(11) Veröffentlichungsnummer: **0 231 003 B1**

(12) # EUROPÄISCHE PATENTSCHRIFT

(45) Veröffentlichungstag der Patentschrift: **07.04.93**

(51) Int. Cl.[5]: **C07C 255/42**, C07D 317/58, C07D 319/08, C07D 321/12, A61K 31/275

(21) Anmeldenummer: **87100995.7**

(22) Anmeldetag: **24.01.87**

Die Akte enthält technische Angaben, die nach dem Eingang der Anmeldung eingereicht wurden und die nicht in dieser Patentschrift enthalten sind.

(54) **Basisch substituierte Phenylacetonitrile, ihre Herstellung und diese enthaltende Arzneimittel.**

(30) Priorität: **31.01.86 DE 3603032**

(43) Veröffentlichungstag der Anmeldung: **05.08.87 Patentblatt 87/32**

(45) Bekanntmachung des Hinweises auf die Patenterteilung: **07.04.93 Patentblatt 93/14**

(84) Benannte Vertragsstaaten: **AT BE CH DE ES FR GB IT LI NL SE**

(56) Entgegenhaltungen:
**DE-A- 1 593 921**
**DE-A- 2 059 985**
**DE-C- 1 154 810**

**ARZNEIMITTELFORSCHUNG, Band 31(I), Nr. 5, 1981, Seite 775, Tabelle 2**

(73) Patentinhaber: **BASF Aktiengesellschaft**
**Carl-Bosch-Strasse 38**
**W-6700 Ludwigshafen(DE)**

(72) Erfinder: **Unger, Liliane, Dr.**
**Waltraudenstrasse 14**
**W-6700 Ludwigshafen(DE)**

Erfinder: **Raschack, Manfred, Dr.**
**Donnersbergstrasse 7**
**W-6714 Weisenheim/Sand(DE)**
Erfinder: **Baldinger, Verena, Dr.**
**Schiffsgasse 6**
**W-6900 Heidelberg(DE)**
Erfinder: **Dengel, Ferdinand, Dr.**
**Am Hirschwald 15**
**W-6916 Wilhelmsfeld(DE)**
Erfinder: **Ehrmann, Oskar, Dr.**
**Reiterweg 19 a**
**W-6800 Mannheim(DE)**
Erfinder: **Treiber, Hans Joerg, Dr.**
**Sperberweg 1**
**W-6835 Bruehl(DE)**
Erfinder: **Seitz, Werner, Dr.**
**Bismarckstrasse 22 b**
**W-6831 Plankstadt(DE)**

EP 0 231 003 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue basisch substituierte Phenylacetonitrile, deren Herstellung sowie Arzneimittel, welche diese Substanzen enthalten.

In der DE-PS 1 154 810, DE-PS 1 493 904, DE-OS 1 593 921, DE-OS 1 643 429, EP-OS 147 707 und EP-OS 64 158 sind basisch substituierte Phenylacetonitrile beschrieben. Aufgrund ihrer calciumantagonistischen Wirkung haben sich Verapamil und Gallopamil in der Therapie der koronaren Herzkrankheit und des Bluthochdrucks bewährt. Weiterhin ist bekannt, daß die beiden Wirkstoffe eine Antiserotoninwirkung aufweisen, die jedoch im Vergleich zu bekannten Serotoninantagonisten schwach ausgeprägt ist [J.E. Taylor u. F.V. DeFeudis, Eur. J. Pharmacol. 106, 215-216 (1985): M. Auguet, F.V. Defeudis und F. Clostre, Neurochem. Int. Vol. 6 (5), 701-710 (1984)].

Es ist bekannt, daß Serotonin an der Entstehung von Spasmen zentraler und peripherer Gefäße sowie bei der zu Gefäßverschlüssen führenden Blutplättchenaggregation beteiligt ist und dabei je nach klinischem Bild die Bedeutung der durch Calcium-Ionen bedingten Erregung sogar übertreffen kann. Bei solchen Erkrankungen finden daher sowohl Calcium-Antagonisten als auch Serotonin-Antagonisten Verwendung. Es ist deshalb von besonderem Wert, beide Wirkprinzipien in einer molekularen Struktur vereint zu haben.

Es wurden nun neue Verbindungen gefunden, deren Antiserotoninwirkung so stark ausgeprägt ist, daß sie die Wirkstärke bekannter Serotoninantagonisten erreichen, die Vergleichsverbindungen Verapamil und Gallopamil aber um ein Vielfaches übertreffen. Die calciumantagonistische Wirkung der neuen Verbindungen ist dabei voll erhalten.

Die Erfindung betrifft neue basisch substituierte Phenylacetonitrile der Formel I

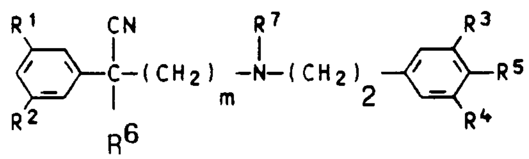

I,

worin $R^1$ und $R^2$ gleich oder verschieden sind und Wasserstoffatome, Halogenatome, $C_1$-$C_3$-Alkyl-, Methoxy- oder Ethoxygruppen bedeuten,

$R^3$, $R^4$ gleich oder verschieden sind und Wasserstoffatome, Halogenatome, Trifluormethyl-, $C_1$-$C_3$-Alkyl-, Methoxy- oder Ethoxygruppen bedeuten,

$R^5$ ein Wasserstoff- oder Halogenatom oder eine $C_1$-$C_3$-Alkylgruppe bedeutet,

wobei mindestens einer der Reste $R^1$ bis $R^5$ ungleich Wasserstoff sein muß und, falls $R^6$ ein Isopropylrest, $R^7$ eine Methylgruppe und m = 3 sind, $R^1$ bis $R^4$ nicht gleichzeitig Methoxy- oder Ethoxygruppen bedeuten können,

$R^6$ ein gesättigter Kohlenwasserstoff mit bis zu 6 C-Atomen oder ein Phenylrest ist,

$R^7$ einen $C_1$-$C_4$-Alkylrest und

m die Zahlen 2 bis 4 bedeuten,

sowie deren Antipoden und Salze mit physiologisch verträglichen Säuren.

Schließlich betrifft die Erfindung die Verwendung von Verbindungen der Formel I sowie deren Antipoden und Salzen mit physiologisch verträglichen Säuren zur Herstellung von Arzneimitteln zur Behandlung von zerebrovaskulären Krankheiten, Hypertonie, koronarer Herzkrankheit, Thrombocytenaggregation und peripherer vaskulärer Erkrankungen.

Als physiologisch verträgliche Säuren kommen z.B. in Frage: Salzsäure, Schwefelsäure, Phosphorsäure, Essigsäure, Malonsäure, Bernsteinsäure, Fumarsäure, Maleinsäure, Zitronensäure, Weinsäure, Milchsäure, Amidosulfonsäure.

Als Halogenatome für $R^1$-$R^5$ kommen bevorzugt Fluor- und Chloratome in Betracht.

Besonders interessant sind die folgenden Verbindungen und deren Enantiomere:

(RS)-5-[(3,5-Dimethoxyphenethyl)methylamino]-2-(3-methoxyphenyl)-2-isopropyl-valeronitril,
(RS)-5-[(3-Methoxyphenethyl)methylamino]-2-(3,5-dimethoxyphenyl)-2-isopropyl-valeronitril,
(RS)-5-[(3-Methoxyphenethyl)methylamino]-2-(3-methoxyphenyl)-2-isopropyl-valeronitril,
(RS)-5-[(3,4-Xylylethyl)methylamino]-2-(3,5-dimethoxyphenyl)-2-isopropyl-valeronitril,
(RS)-5-[(Phenethyl)methylamino]-2-(3,5-dimethoxyphenyl)-2-isopropyl-valeronitril.

Die neuen Verbindungen werden hergestellt, indem man

a) Phenylacetonitrile der Formel II

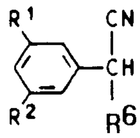

II,

worin $R^1$, $R^2$ und $R^6$ die angegebene Bedeutung haben, mit 1-Phenylazaalkanen der Formel III

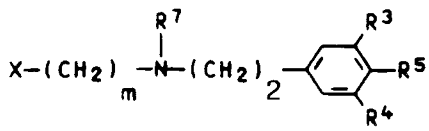

III,

worin $R^3$-$R^5$, $R^7$ und m die angegebene Bedeutung besitzen, und X eine Austrittsgruppe darstellt, umsetzt oder

b) basisch substituierte Phenylacetonitrile der Formel IV

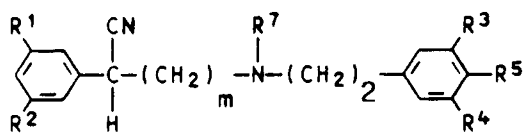

IV,

worin $R^1$-$R^5$, $R^7$ und m die angegebene Bedeutung haben mit Verbindungen der Formel V

$R^6$-Y V,

worin $R^6$ die angegebene Bedeutung hat und Y eine Austrittsgruppe darstellt, zur Reaktion bringt oder

c) Phenylacetonitrile der Formel VI

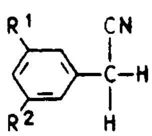

VI,

worin $R^1$ und $R^2$ die angegebenen Bedeutungen haben, mit 1-Phenylazaalkanen der Formel III und Verbindungen der Formel V umsetzt oder

d) Phenylacetonitrile der Formel VII

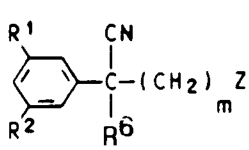

VII,

worin $R^1$, $R^2$, $R^6$ und m die oben angegebenen Bedeutungen haben und Z eine Austrittsgruppe darstellt, mit einem Phenylalkylamin der Formel VIII

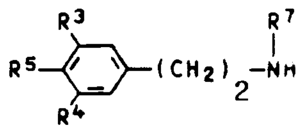 VIII,

worin $R^3$-$R^5$ und $R^7$ die obige Bedeutung besitzen, zur Reaktion bringt oder
e) Phenylacetonitrile der Formel IX

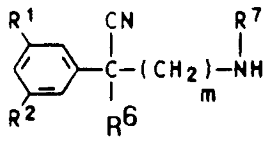 IX,

worin $R^1$, $R^2$, $R^6$, $R^7$ und m die beschriebene Bedeutung haben, mit Phenylalkyl-derivaten der Formel X

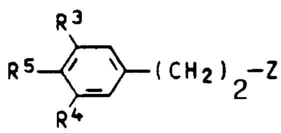 X,

worin $R^3$, $R^4$, $R^5$ und Z die oben angegebene Bedeutung haben, zur Reaktion bringt oder
f) basisch substituiere Phenylaceonitrile der Formel XI

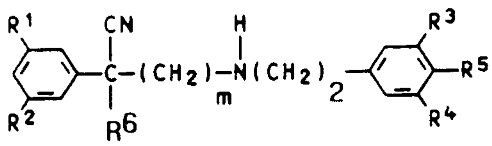 XI,

worin $R^1$-$R^6$ und m die angegebene Bedeutung haben, alkyliert oder
g) Phenylacetonitrile der Formel IX mit Aldehyden der Formel XII

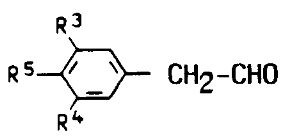 XII

worin $R^3$-$R^5$ dasselbe wie oben bedeuten, unter reduktiven Bedingungen umsetzt oder
h) Aldehyde der Formel XIII

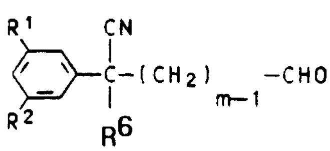 XIII

worin $R^1$, $R^2$, $R^6$ und m dasselbe wie oben bedeuten, mit Phenylalkylaminen der Formel VIII unter reduktiven Bedingungen umsetzt, oder

i) Dinitrile der Formel XIV

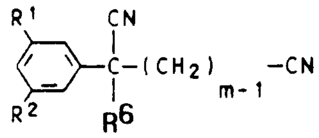

XIV

worin $R^1$, $R^2$ und m dasselbe wie oben bedeuten in Gegenwart eines Phenalkylamins der Formel VIII reduziert oder k) Nitrile der Formel XV

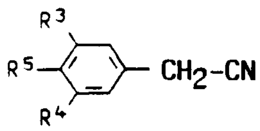

XV

worin $R^3$ bis $R^5$ dasselbe wie oben bedeuten in Gegenwart eines Phenylacetonitrils der Formel IX reduziert und die erhaltenen Verbindungen gewünschtenfalls in ihre Antipoden trennt und/oder in ein Salz mit einer physiologisch verträglichen Säure überführt.

Die Umsetzung a) kann beispielsweise durchgeführt werden, indem man ein CH-acides Phenylacetonitril der Formel II in einem inerten Lösungsmittel mit einer Base metalliert und anschließend mit Verbindungen der Formel III umsetzt. Gegebenenfalls kann auch so verfahren werden, daß die Base zu einer Lösung von Verbindungen der Formel II und III zugegeben wird.

Als Basen kommen in Betracht: Alkalimetall-hydride, -hydroxide, -alkoholate, -amide und metallorganische Verbindungen. Vorzugsweise werden verwendet: Natriumamidpulver und -suspension, Kaliumhydroxidpulver, Butyllithium, Lithiumdiisopropylamid.

Als Lösungsmittel für die Reaktion eignen sich aromatische und aliphatische Kohlenwasserstoffe, jedoch sind auch höhersiedende aliphatische Ether und dipolar aprotische Lösungsmittel geeignet. Bevorzugt wird mit Toluol gearbeitet.

Reaktion a) kann auch nach einem phasentransferkatalysierten Verfahren durchgeführt werden. Als Katalysatoren finden quartäre Ammonium-, Phosphoniumsalze, Kronenether, Polyethylenglykol-dialkylether (z.B. PEG 600-dibutylether) und Tris-(3,6-dioxaheptyl)amin (TDA-1) Verwendung.

Die Reaktionstemperaturen sind von den eingesetzten Basen abhängig, z.B. wird mit Butyllithium bei Temperaturen zwischen 0 und -100 ° C und bei Verwendung von Natriumamid vorzugsweise bei 50 ° C bis 150 ° C gearbeitet.

Die Umsetzung von Verbindungen der Formel IV zu den erfindungsgemäßen Verbindungen (Verfahren b) erfolgt in ähnlicher Weise wie bei Verfahren a).

Als Reaktionspartner der Formel IV kommen beispielsweise in Frage: Alkanderivate mit einer Austrittsgruppe, wie Halogenide, Schwefelsäureester, Tosylate, Mesylate oder Triflate.

Bei Verfahren c) kann die Reihenfolge der Zugabe der Verbindungen III, V und VI beliebig gewählt und auf eine Isolierung von Zwischenprodukten verzichtet weden.

Die Reaktion d) erfolgt durch einfaches Erhitzen der Reaktionspartner auf vorzugsweise 120 bis 180 ° C. Sie kann auch in einem Lösungmsittel erfolgen, doch ist dieses nicht erforderlich. Dasselbe gilt für die Umsetzung e). Als Z eignet sich in beiden Fällen Halogen, vorzugsweise Chlor und Brom.

Die Alkylierung f) gelingt mit Dialkylsulfaten oder Alkylhalogeniden in Gegenwart eines Säurefängers, wie Triethylamin oder Kaliumcarbonat. Zweckmäßigerweise arbeitet man in einem Lösungsmittel, wie Toluol oder in einem dipolar aprotischen Lösungsmittel, wie Dimethylformamid. Die Methylierung kann auch nach Leuckart-Wallach mit Formaldehyd/Ameisensäure durchgeführt werden.

Beim Verfahren g) und h) werden die beiden Reaktionspartner der Formel IX und XII bzw. XIII und VIII in einer Kondensation unter reduktiven Bedingungen umgesetzt.

Als Lösungsmittel sind aliphatische und aromatische Kohlenwasserstoffe, halogenierte Kohlenwasserstoffe, Ether, Alkohole oder niedrige Fettsäuren geeignet. Die Reaktionstemperaturen liegen zwischen 0 und 150 ° C, vorzugsweise 20 bis 70 ° C.

Als Reduktionsmittel kommen in Betracht: Wasserstoff in Gegenwart eines Katalysators, z.B. $PtO_2$, Pd/C, Nickel- oder Kobalt-Katalysatoren, nascierender Wasserstoff, den man aus Metall und Säure erhält, komplexe Metallhydride (z.B. $NaBH_4$) oder Hydriddonatoren (z.B. Ameisensäure).

Die Reduktion des Dinitrils der Formel XIV in Gegenwart eines Phenalkylamins der Formel VIII (Umsetzung i) bzw. eines Nitrils der Formel XV in Gegenwart eines Phenylacetonitrils der Formel IX (Umsetzung k) erfolgt vorzugsweise als katalytische Hydrierung mit einem Edelmetallkatalysator, vorzugsweise Pd/C. Die Reaktionstemperaturen liegen zwischen 30 und 80 °C, vorzugsweise bei 60 °C. Die Reaktion kann unter Normaldruck oder Überdruck bis 6 bar erfolgen. Als Lösungsmittel werden niedrige Alkohole, Essigsäure oder aromatische Kohlenwasserstoffe, vorzugsweise niedrige aliphatische Alkohole, wie Ethanol, verwendet. Bezogen auf das eingesetzte Amin sind 0,1 bis 10 Gew.% Pd/C-Katalysator erforderlich, wobei der Katalysator 1 bis 10 Gew.% Pd auf Kohlenstoff enthält.

Wird die Reduktion in Gegenwart eines Katalysators durchgeführt, so arbeitet man bevorzugt bei Atmosphärendruck.

Die Reaktionen a) bis i) sind beschrieben in DE-PS 11 54 810, DE-PS 11 58 083, DE-PS 20 59 923, DE-AS 22 63 527, DE-PS 26 31 222, DE-OS 30 34 221 und EP-OS 165 322.

Die neuen Verbindungen besitzen mindestens ein asymmetisches Kohlenstoffatom. Folglich können sie entweder in Form reiner Enantiomeren oder als racemische Mischungen hergestellt werden, vgl. DE-PS 20 59 923 und DE-PS 20 59 985. Die Racemate der Verbindungen I können durch herkommliche Techniken, z.B. durch Trennung (fraktionierte Kristallisation, Säulenchromatographie) der diastereomeren Salze, in ihre optischen Anti poden gespalten werden. Die diastereomeren Salze sind durch Umsetzung der Verbindungen I mit chiralen Säuren herstellbar. Die Enantiomeren können auch durch die Verwendung optisch aktiver Ausgangsverbindungen erhalten werden.

Die bislang nicht beschriebenen Ausgangsstoffe der Formeln IV, VII, IX, X, XI, XIII und XIV lassen sich wie folgt herstellen:

Durch Umsetzen von Phenylacetonitrilen (VI) mit 1-Phenyl-omega-halogen-azaalkanen (vgl. III) in Gegenwart von Natriumamid in Toluol erhalt man die Verbindungen IV.

Die Verbindungen VII lassen sich durch Umsetzen von 1-Cyan-1-phenyl-alkanen (II) mit Chlorpropanol und anschließende Reaktion des so erhaltenen Alkohols mit beispielsweise Thionylchlorid darstellen.

Die Verbindungen IX gewinnt man aus den Phenylacetonitrilderivaten VII durch Umsetzen mit Alkylaminen.

Die Substanzen der Formel X werden hergestellt, indem man z.B. die Phenylacetonitrile VI zu Phenylessigsäure hydrolysiert, letztere zum entsprechenden Alkohol reduziert und diesen beispielsweise mit Thionylchlorid chloriert.

Die Umsetzung von 1-Cyan-1-phenyl-alkanen (II) mit 1-Phenyl-omega-halogen-aza-alkanen (vgl. III) in Gegenwart von Natriumamid in Toluol führt zu den Verbindungen XI.

Durch Umsetzung von 1-Cyan-1-phenyl-alkanen (II) mit omega-Halogen-aldehyddiethylacetalen in Gegenwart von Natriumamid in Toluol und anschließende Behandlung des so erhaltenen Reaktionsproduktes mit Säure gewinnt man die Verbindungen XIII.

Dinitrile der Formel XIV sind durch Addition von Acrylnitril an Phenylacetonitrile der Formel II zugänglich.

Die Verbindungen besitzen wertvolle pharmakologische Eigenschaften und sind daher geeignet zur Behandlung von Herz-/Kreislauferkrankungen, insbesondere zerebrovaskulären Krankheiten - wie beispielsweise Migräne, Vasospasmen, Subarachnoidalblutung, apoplektischem Insult, zerebraler Ischämie - ferner bei peripheren vaskulären Erkrankungen - wie Raynaud's Krankheit -, koronarer Herzkrankheit, Hypertonie und Kreislaufschock.

Die überlegene Wirkung der Verbindungen läßt sich beispielsweise durch die Hemmung der durch Serotonin induzierten Blutdrucksteigerung an der despinalisierten Ratte bei intravenöser Applikation zeigen:

In Amobarbitalnarkose (120 mg/kg Körpergewicht i.p.) werden bei 220 bis 280 g schweren männlichen Sprague-Dawley-Ratten die A.carotis und die V.jugularis kanüliert, beidseitig Vagus und Sympathikus durchtrennt und die Tiere an eine Atempumpe angeschlossen. Die Despinalisierung erfolgt mittels eines Stabes durch die Orbita. Injektionen von 0,0215 mg/kg Serotonin i.v. steigern bei unvorbehandelten Kontrolltieren den arteriellen Mitteldruck von 53 ± 0,6 mmHg um 80 ± 1,2 mmHg (n = 95). Kriterium einer Substanzwirkung ist die relative Hemmung (Δ %) der durch Serotonin ausgelösten Blutdrucksteigerung (Δ mmHg). Die Auswertung erfolgt quantitativ als Analyse der linearen Regression ($y = a + b x$) zwischen log Dosis (mg/kg) und rel. Hemmung (Δ %) der Serotonin-Blutdrucksteigerung. Für Vergleiche wurden als ED 50 % die Dosen berechnet, welche die Serotoninblutdrucksteigerung um 50 % hemmen.

So wurden folgende Werte erhalten:

| Substanz des Beispiels | ED 50 % |
|---|---|
| 1 | 0,0464 |
| 2 | 0,0730 |
| 3 | 0,0464 |
| 4 | 0,0318 |
| 5 | 0,0464 |
| 6 | 0,1 |
| 7 | 0,1 |
| 8 | 0,0499 |
| 11 | 0,0464 |
| 12 | 0,0464 |
| 15 | 0,1 |
| 17 | 0,1 |
| 18 | 0,1 |
| 21 | 0,1 |
| 25 | 0,0464 |
| Verapamil | 0,363 |
| Gallopamil | > 0,1 (22 %) |

Die erfindungsgemäßen Verbindungen wirken an despinalisierten Ratten nach i.v. Applikation bis 11mal stärker serotoninantagonistisch als Verapamil.

Die Verbindungen können in üblicher Weise oral oder parenteral gegeben werden. Die Dosierung hängt vom Alter, Zustand und Gewicht des Patienten sowie von der Applikationsart ab. In der Regel beträgt die tägliche Wirkstoffdosis zwischen etwa 0.1 und 10 mg/kg Körpergewicht bei oraler Gabe und zwischen 0,01 und 1,0 mg/kg Körpergewicht bei parenteraler Gabe. Im Normalfall werden tägliche Dosen von 1 bis 5 mg/kg oral und 0,05 bis 0,25 mg/kg parenteral angewendet.

Die erfindungsgmäßen Wirkstoffe können in die üblichen galenischen Anwendungsformen, wie Tabletten, Dragees, Lösungen, Emulsionen, Pulver. Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffektes, wie Carboxipolymethylen, Carboximethylcellulose, Celluloseacetatphthalat oder Polyvinylacetat erhalten werden.

Die Tabletten können auch aus mehreren Schichten bestehen. Entsprechend können Dragees durch Überziehen von analog den Tabletten hergestellten Kernen mit üblicherweise in Dragee-Überzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmacksverbesserndes Mittel, z.B. Aromastoffe, wie Vanillin oder Orangenextrakt, enthalten.

Sie können außerdem Suspendierhilfsstoffe oder Dickungsmittel, wie Natriumcarboximethylcellulose, Netzmittel, beispielsweise Kondensationsprodukte von Fettalkoholen mit Ethylenoxid oder Schutzstoffe, wie p-Hydroxibenzoate, enthalten.

Injektionslösungen werden in üblicher Weise, z.B. unter Zusatz von Konservierungsmitteln, wie p-Hydroxibenzoaten oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyethylenglykol bzw. dessen Derivaten, herstellen.

Die folgenden Beispiele erläutern die Erfindung ohne sie zu beschränken.

Beispiel 1

5-[(3,5-Dimethoxyphenethyl)methylamino]-2-(3-methoxyphenyl)-2-isopropyl-valeronitril

Eine Lösung von 18,9 g (0,1 Mol) α-Isopropyl-3-methoxyphenylacetonitril und 27,2 g (0,1 Mol) N-(3-Chlorpropyl)-N-methyl-3,5-dimethoxyphenethylamin in 100 ml Toluol wurde unter Rühren auf 85°C erwärmt. Anschließend wurden innerhalb von 2 h 8 g (ca. 0,1 Mol) 50 %ige toluolische Natriumamidsuspension zugetropft. Die Reaktionslösung wurde 15 min bei 85°C nachgerührt und nach dem Erkalten mit 200 ml Eiswasser versetzt. Die Toluolphase wurde abgetrennt, zweimal mit Wasser gewaschen und anschließend das Toluol im Vakuum abdestilliert. Der ölige Rückstand wurde in 150 ml Ethanol gelöst und mit 10 g Oxalsäure versetzt. Das ausgefallene Hydrogenoxalat wurde abgesaugt und aus 200 ml Ethanol umkristallisiert. Man isolierte 43,4 g (85 %) Hydrogenoxalat, Fp. 159 bis 161°C.

Beispiel 2

5-[(3-Methoxyphenethyl)methylamino]-2-(3,5-dimethoxyphenyl)-2-isopropyl-valeronitril

38,3 g (0,1 Mol) 5-[(3-Methoxyphenethyl)methylamino]-2-(3,5-dimethoxyphenyl)-valeronitril wurden in 200 ml Toluol gelöst und mit 4,7 g (0,12 Mol) pulverisiertem Natriumamid 1 h unter Rühren und Rückfluß erhitzt. Anschließend tropfte man innerhalb von 60 min eine Lösung von 15,7 g (0,12 Mol) Isopropylbromid in 30 ml Toluol zu und erhitzte noch 2 h unter Rückfluß. Die erkaltete Reaktionsmischung wurde in Wasser gegossen, die Toluolphase mehrmals mit Wasser gewaschen und anschließend das Toluol abdestilliert. Der Rückstand wurde in 100 ml Isopropanol gelöst und mit ethanolischer Salzsäure versetzt. Das Hydrochlorid wurde aus Isopropanol umkristallisiert. Man isolierte 46,9 g (92 %) Hydrochlorid, Fp. 141 bis 143°C.

Beispiel 3

5-[(Phenethyl)methylamino]-2-(3-methoxyphenyl)-2-isopropyl-valeronitril

13,5 g (0,1 Mol) 3-Methoxyphenylacetonitril wurden in 15 ml Toluol gelost und mit 52 g (0,8 Mol) 85 %igem Kaliumhydroxidpulver und 0,2 g Tris-(3,6-dioxaheptyl)amin versetzt. Anschließend wurden unter Rühren 12,3 g (0,1 Mol) Isopropylbromid so zugetropft, daß die Reaktionstemperatur 50°C nicht überstieg. Nach beendeter Zugabe wurde 2 h bei 50°C nachgerührt und anschließend eine Lösung von 21,2 g (0,1 Mol) N-(3-Chlorpropyl)-N-methyl-phenethylamin in 20 ml Toluol bei 90°C zugegeben. Die Reaktionsmischung wurde weitere 3 h bei 90°C gerührt, nach Erkalten mit 100 ml Wasser versetzt und die Toluolphase abgetrennt. Nach Abdestillieren des Toluols erhielt man ein gelbes Öl, das über eine Kieselgelsäule mit einem Lösungsmittelgemisch aus Methylenchlorid/Aceton/Methanol (9/1/0,5) chromatographiert wurde. Die chromatographisch reine Base wurde in Essigester gelöst und mit ethanolischer Salzsäure das Hydrochlorid ausgefällt. Man erhielt 26,1 g (65 %), Fp. 154 bis 156°C.

Beispiel 4

5-[(3-Methoxyphenethyl)methylamino]-2-(3-methoxyphenyl)-2-isopropyl-valeronitril

47,1 g (0,1 Mol) [(3-Methoxyphenethyl)amino]-2-(3-methoxyphenyl)-2-isopropyl-valeronitril wurden in der Kälte in 50 ml Ameisensäure gelost und nach Zugabe von 11,9 ml 35 %iger wäßriger Formalinlösung (0,15 Mol) bis zur Beendigung der Kohlendioxidentwicklung auf dem Wasserbad erhitzt. Nach dem Abkühlen wurde die Reaktionslosung mit Wasser verdünnt, durch Zugabe von Ammoniak alkalisch gemacht und mit Ether die abgeschiedene Base extrahiert. Die etherische Lösung wurde mehrmals mit Wasser gewaschen, mit Kaliumcarbonat getrocknet und anschließend der Ether abdestilliert. Der Rückstand wurde in Essigester gelöst und mit ethanolischer Salzsäure versetzt. Es fielen 37,9 g (88 %) Hydrochlorid aus, Fp. 102 bis 104°C.

Beispiel 5

5-[(Phenethyl)methylamino]-2-(3,5-dimethoxyphenyl)-2-isopropyl-valeronitril

29,5 g (0,1 Mol) α-Isopropyl-α-(3-chlorpropyl)-3,5-dimethoxyphenylacetonitril und 13,5 g (0,1 Mol) N-Methyl-phenethylamin wurden in 45 ml Hexamethylphosphorsäuretriamid gelöst und mit 30 g wasserfreiem gepulvertem Kaliumcarbonat versetzt. Das Reaktionsgemisch wurde 4 h auf 80 °C erhitzt, nach Abkühlen mit 500 ml Wasser versetzt und zweimal mit Ether extrahiert. Die Etherphase wurde mehrmals mit Wasser gewaschen, über Kaliumcarbonat getrocknet und der Ether abdestilliert. Die erhaltene Base wurde in das Hydrochlorid überführt. Man erhielt 34,8 g (81 %), Fp. 126 bis 127 °C.

Beispiel 6

5-[(4-Chlorphenethyl)methylamino]-2-(3,5-dimethoxyphenyl)-2-isopropyl-valeronitril

Analog Beispiel 5 wurden aus 29,0 g (0,1 Mol) 5-Methylamino-2-(3,5-dimethoxyphenyl)-2-isopropyl-valeronitril und 21,0 g (0,12 Mol) 4-Chlorphenethylchlorid nach chromatographischer Reinigung 27,9 g (60 %) Base isoliert, die in das Amidosulfonat überführt wurde, Fp. 122 bis 123 °C.

Beispiel 7

5-[(3,4-Dichlorphenethyl)methylamino]-2-(3,5-dimethoxyphenyl)-2-isopropyl-valeronitril

27,5 g (0,1 Mol) 4-(3,5-Dimethoxyphenyl)-4-cyano-5-methyl-hexanal und 20,4 g (0,1 Mol) N-Methyl-3,4-dichlorphenethylamin wurden in 100 ml Toluol gelöst. Dazu gab man in der Kälte 4,6 g (0,1 Mol) Ameisensäure. Das Reaktionsgemisch wurde bis zum Abklingen der Gasentwicklung unter Rückfluß erhitzt.

Die erkaltete Reaktionslösung wurde mit wäßriger Kaliumcarbonatlösung versetzt, das freigesetzte Amin mit Ether extrahiert und die etherische Phase mehrmals mit Wasser gewaschen. Nach Trocknen und Abdestillieren des Ethers wurde die ölige Base in Isopropanol gelöst und mit ethanolischer Salzsäure versetzt. Man erhielt nach Umkristallisieren aus Isopropanol 46 g (92 %) 5-[(3,4-Dichlorphenethyl)-methylamino]-2-(3,5-dimethoxyphenyl)-2-isopropyl-valeronitril-hydrochlorid, Fp. 147 bis 148,5 °C.

Beispiel 8

5-[(3-Trifluormethylphenethyl)methylamino]-2-(3,5-dimethoxyphenyl)-2-isopropyl-valeronitril

Ein Gemisch aus 29,0 g (0,1 Mol) 2-(3,5-Dimethoxyphenyl)-2-isopropyl-5-methylamino-valeronitril (hergestellt durch Umsetzung von α-Isopropyl-3,5-dimethoxyphenylacetonitril und 3-Methyl-formamido-1-chlorpropan in Gegenwart von Natriumhydrid und anschließende Abspaltung der Formylgruppe mittels Salzsäure) und 18,8 g (0,1 Mol) 3-Trifluormethylphenylacetaldehyd wurde mit 400 mg 5 %igem Palladium auf Kohle in 100 ml Toluol katalytisch unter Atmosphärendruck bei 25 bis 30 °C 20 h reduziert. Nach Entfernung des Katalysators wurde die toluolische Lösung mehrmals mit Wasser gewaschen, mit Kaliumcarbonat getrocknet und anschließend das Toluol abdestilliert. Der Rückstand wurde in Isopropanol gelöst und mit ethanolischer Salzsäure versetzt. Nach Kristallisation aus Isopropanol erhielt man 39,9 g (80 %) 5-[-(3-Trifluormethylphenethyl)methylamino]-2-(3,5-dimethoxyphenyl)-2-isopropyl-valeronitril, Fp. 136 bis 138 °C.

Beispiel 9

5[(3,5-Dimethoxyphenethyl)methylamino]-2-(3,5-dimethoxyphenyl-2-(n--propyl)-valeronitril

In einer Hydrierapparatur werden 40,8 (0,15 Mol) 4-(3,5-Dimethoxyphenyl)-4-cyanoheptansäurenitril in 170 ml Isopropanol gelöst, mit 7 g 5 %igem Pd/C versetzt und mit Stickstoff gespült. Nach Zugabe von 2 ml wäßriger Cetyl-trimethylammoniumhydroxid-Lösung wurde unter kräftigem Rühren ein Wasserstoffstrom durch die Apparatur geleitet und das Vorratsgefäß mit Wasserstoff gefüllt. Dann gab man 29,3 g (0,15 Mol) N-Methyl-3,5-dimethoxyphenethylamin zu dem Reaktionsgemisch und hydrierte bei 50 bis 60 °C unter leichtem Wasserstoffüberdruck. Nach 2 h war die Hydrierung beendet.

Nach Abtrennen des Katalysators wurde die Isopropanollösung im Vakuum eingeengt. Man nahm den Rückstand in Ether auf und schüttelte mehrmals mit Kochsalzlösung. Anschließend wurde die Base mit wäßriger Amidosulfonsäurelösung der etherischen Phase entzogen. Die saure Lösung wurde mehrmals mit Ether ausgeschüttelt, anschließend wurde mit wäßriger Kaliumcarbonatlösung die Base freigesetzt. Die Base wurde säulenchromatographisch gereinigt.

| | | | | |
|---|---|---|---|---|
| Analyse: | ber.<br>gef. | C 71,3<br>C 71,5 | H 8,4<br>H 8,5 | N 6,2<br>H 6,1 |

Beispiel 10

5-[(3,5-Dimethoxyphenethyl)methylamino]-2-(3,5-dimethoxyphenyl)-2-phenylvaleronitril

Analog Beispiel 9 wurden aus 32,4 g (0,1 Mol) 5-Methylamino-2-(3,5-dimethoxyphenyl)-2-phenylvaleronitril und 17,7 g (0,1 Mol) 3,5-Dimethoxyphenylacetonitril 41,6 g (85 %) Base isoliert, die säulenchromatographisch gereinigt wurde.

| | | | | |
|---|---|---|---|---|
| Analyse: | ber.<br>gef. | C 73,7<br>C 73,6 | H 7,4<br>H 7,5 | N 5,7<br>N 5,7 |

In analoger Weise erhielt man:

11) 5-[(3-Methoxyphenethyl)methylamino]-2-phenyl-2-isopropyl-valeronitril

| | | | | |
|---|---|---|---|---|
| Analyse: | ber.<br>gef. | C 79,1<br>C 79,0 | H 8,9<br>H 9,0 | N 7,7<br>N 7,7 |

12) 5-[(3,5-Dimethoxyphenethyl)methylamino]-2-phenyl-2-isopropyl-valeronitril-aminosulfonat, Fp. 119 bis 120 ° C

13) 5-[(3-Chlorphenethyl)methylamino]-2-(3,5-dimethoxyphenyl)-2-isopropyl-valeronitril

| | | | | | |
|---|---|---|---|---|---|
| Analyse: | ber.<br>gef. | C 70,0<br>C 70,1 | H 7,8<br>H 7,7 | N 6,5<br>N 6,5 | Cl 8,3<br>Cl 8,2 |

14) 5-[(3,5-Dichlorphenethyl)methylamino]-2-(3,5-dimethoxyphenyl)-2-isopropyl-valeronitril

| | | | | | |
|---|---|---|---|---|---|
| Analyse: | ber.<br>gef. | C 64,8<br>C 64,9 | H 7,0<br>H 6,9 | N 6.0<br>N 6,0 | Cl 15,3<br>Cl 15,4 |

15) 5-[(3,5-Dimethoxyphenethyl)methylamino]-2-(3-ethoxyphenyl)-2-isopropyl-valeronitril-hydrochlorid, Fp. 124-126 ° C

16) 5-[(3-Ethoxyphenethyl)methylamino]-2-(3,5-dimethoxyphenyl)-2-isopropyl-valeronitril-hydrochlorid, Fp. 113-115 ° C

17) 5-[(3,5-Diethoxyphenethyl)methylamino]-2-(3,5-dimethoxyphenyl)-2-isopropyl-valeronitril-hydrochlorid, Fp. 117-119 ° C

18) 5-[(3,4-Xylylethyl)methylamino]-2-(3,5-dimethoxyphenyl)-2-isopropyl-valeronitril-hydrochlorid, Fp. 136,5-138,5 ° C

19) 5-[(3,5-Xylylethyl)methylamino]-2-(3,5-dimethoxyphenyl)-2-isopropyl-valeronitril

| | | | | |
|---|---|---|---|---|
| Analyse: | ber.<br>gef. | C 76,7<br>C 76,5 | H 9,1<br>H 9,0 | N 6,6<br>N 6,5 |

20) 5-[(3,5-Dimethoxyphenethyl)methylamino]-2-(3,5-xylyl)-2-isopropyl-valeronitril

| | | | | |
|---|---|---|---|---|
| Analyse: | ber.<br>gef. | C 76,7<br>C 76,9 | H 9,1<br>H 9,2 | N 6,6<br>N 6,6 |

21) 5-[(3,5-Diethoxyphenethyl)methylamino]-2-phenyl-2-isopropyl-valeronitril

| | | | | |
|---|---|---|---|---|
| Analyse: | ber.<br>gef. | C 76,7<br>C 76,8 | H 9,1<br>H 9,0 | N 6,6<br>N 6,5 |

22) 5-[(3-Methoxyphenethyl)methylamino]-2,2-diphenyl-valeronitril

| | | | | |
|---|---|---|---|---|
| Analyse: | ber.<br>gef. | C 81,4<br>C 81,2 | H 7,6<br>H 7,7 | N 7,0<br>N 6,9 |

23) 5-[(3,5-Dimethoxyphenethyl)methylamino]-2,2-diphenyl-valeronitril

| | | | | |
|---|---|---|---|---|
| Analyse: | ber.<br>gef. | C 78,5<br>C 78,6 | H 7,5<br>H 7,3 | N 6,5<br>N 6,5 |

24) 5-[(3,4-Dichlorphenethyl)methylamino]-2-(3-ethoxyphenyl)-2-isopropyl--valeronitril-hydrochlorid, Fp. 165-168 ° C

25) 5-[(3,4-Dimethoxyphenethyl)methylamino]-2-(3-ethoxyphenyl)-2-isopropyl-valeronitril,

| | | | | |
|---|---|---|---|---|
| Analyse: | ber.<br>gef. | C 73,9<br>C 73,7 | H 8,7<br>H 8,8 | N 6,4<br>N 6,5 |

26) 5-[(3-Ethoxyphenethyl)methylamino]-2-(3-ethoxyphenyl)-2-isopropyl-valeronitril-hydrochlorid, Fp. 138-140 ° C

27) 5-[(3-Ethoxyphenethyl)methylamino]-2-(3,5-dimethoxyphenyl)-2-isopropyl-valeronitril-hydrochlorid, Fp. 113-115 ° C

28) 5-[(4-Fluorphenethyl)methylamino]-2-(3,5-dimethoxyphenyl)-2-isopropyl-valeronitril-hydrogenoxalat, Fp. 146-148 ° C

29) 5-[(3-Fluor-4-methoxyphenethyl)methylamino]-2-(3,5-dimethoxyphenyl)-2-(3,5-dimethoxyphenyl)-2-isopropyl-valeronitril-hydrochlorid, Fp. 114-117 ° C

30) 5-[(4-Methoxyphenethyl)methylamino]-2-(3,5-dimethoxyphenyl)-2-isopropyl-valeronitril-hydrochlorid, Fp. 128-130 ° C

31) 5-[(3,4-Dimethoxyphenethyl)methylamino]-2-(3,5-dimethoxyphenyl)-2--isopropyl-valeronitril-hydrochlorid, Fp. 162 ° C

32) 5-[(Phenethyl)methylamino]-2-(3-chlorphenyl)-2-isopropyl-valeronitril,

| | | | | |
|---|---|---|---|---|
| Analyse: | ber.<br>gef. | C 74,9<br>C 74,8 | H 7,9<br>H 7,7 | N 7,6<br>N 7,5 |

33) 5-[(4-Fluorphenethyl)methylamino]-2-phenyl-2-isopropyl-valeronitril,

| | | | | |
|---|---|---|---|---|
| Analyse: | ber.<br>gef. | C 78,4<br>C 78,3 | H 8,3<br>H 8,2 | N 8,0<br>N 7,9 |

Beispiel 34

Auf einer Tablettenpresse werden in üblicher Weise Tabletten folgender Zusammensetzung gepreßt:

| | |
|---|---|
| 40 mg | Substanz des Beispiels 3 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister). |

Beispiel 35

In üblicher Weise werden Dragees folgender Zusammensetzung hergestellt:

| | |
|---|---|
| 20 mg | Substanz des Beispiels 3 |
| 60 mg | Kernmasse |
| 60 mg | Verzuckerungsmasse |

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm.Ind. 1962, 586). Die Verzuckerungsmasse besteht aus 5 Teilen Rohzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 36

10 g Substanz des Beispiels 3 werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Patentansprüche**
**Patentansprüche für folgende Vertragsstaaten : BE, CH, DE, FR, GB, IT, LI, NL, SE**

**1.** Basisch substituierte Phenylacetonitrile der Formel I

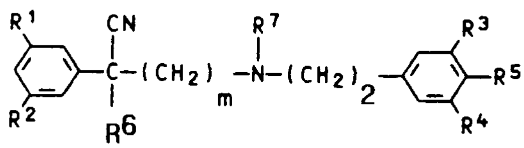

I,

worin $R^1$ und $R^2$ gleich oder verschieden sind und Wasserstoffatome, Halogenatome, $C_1$-$C_3$-Alkyl-, Methoxy- oder Ethoxygruppen bedeuten,
$R^3$, $R^4$ gleich oder verschieden sind und Wasserstoffatome, Halogenatome, Trifluormethyl-, $C_1$-$C_3$-Alkyl-, Methoxy- oder Ethoxygruppen bedeuten,
$R^5$ ein Wasserstoff- oder Halogenatom oder eine $C_1$-$C_3$-Alkylgruppe bedeutet,
wobei mindestens einer der Reste $R^1$ bis $R^5$ ungleich Wasserstoff sein muß und, falls $R^6$ ein Isopropylrest, $R^7$ eine Methylgruppe und m = 3, sind, $R^1$ bis $R^4$ nicht gleichzeitig Methoxy- oder Ethoxygruppen bedeuten können,
$R^6$ ein gesättigter Kohlenwasserstoff mit bis zu 6 C-Atomen oder ein Phenylrest ist,
$R^7$ einen $C_1$-$C_4$-Alkylrest und
m die Zahlen 2 bis 4 bedeuten,
sowie deren Antipoden und Salze mit physiologisch verträglichen Säuren.

**2.** Verbindungen gemäß Anspruch 1 zur Verwendung bei der Bekämpfung von Krankheiten.

**Patentansprüche für folgende Vertragsstaaten : AT, ES**

**1.** Verfahren zur Herstellung basisch substituierter Phenylacetonitrile der Formel I

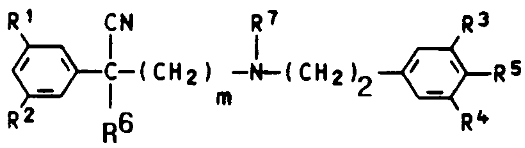

I,

worin $R^1$ und $R^2$ gleich oder verschieden sind und Wasserstoffatome, Halogenatome, $C_1$-$C_3$-Alkyl-, Methoxy- oder Ethoxygruppen bedeuten,
$R^3$, $R^4$ gleich oder verschieden sind und Wasserstoffatome, Halogenatome, Trifluormethyl-, $C_1$-$C_3$-Alkyl-, Methoxy- oder Ethoxygruppen bedeuten,
$R^5$ ein Wasserstoff- oder Halogenatom oder eine $C_1$-$C_3$-Alkylgruppe bedeutet,
wobei mindestens einer der Reste $R^1$ bis $R^5$ ungleich Wasserstoff sein muß und, falls $R^6$ ein Isopropylrest, $R^7$ eine Methylgruppe und m = 3, sind, $R^1$ bis $R^4$ nicht gleichzeitig Methoxy- oder Ethoxygrupen bedeuten können,
$R^6$ ein gesättigter Kohlenwasserstoffrest mit bis zu 6 C-Atomen oder ein Phenylrest ist,
$R^7$ einen $C_1$-$C_4$-Alkylrest und
m die Zahlen 2 bis 4 bedeuten,
sowie deren Antipoden und Salze mit physiologisch verträglichen Säuren, dadurch gekennzeichnet, daß man

a) Phenylacetonitrile der Formel II

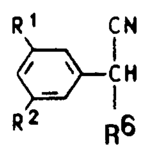

II,

worin $R^1$, $R^2$ und $R^6$ die angegebene Bedeutung haben, mit 1-Phenylazaalkanen der Formel III

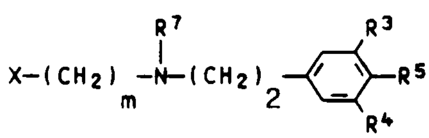

III,

worin $R^3$-$R^5$, $R^7$ und m die angegebene Bedeutung besitzen, und X eine Austrittsgruppe darstellt, umsetzt oder

b) basisch substituierte Phenylacetonitrile der Formel IV

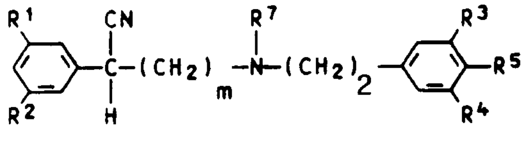

IV,

worin $R^1$-$R^5$, $R^7$ und m die angegebene Bedeutung haben mit Verbindungen der Formel V

$R^6$-Y V,

worin $R^6$ die angegebene Bedeutung hat und Y eine Austrittsgruppe darstellt, zur Reaktion bringt oder
c) Phenylacetonitrile der Formel VI

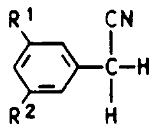

VI,

worin $R^1$ und $R^2$ die angegebenen Bedeutungen haben, mit 1-Phenylazaalkanen der Formel III und Verbindungen der Formel V umsetzt oder
d) Phenylacetonitrile der Formel VII

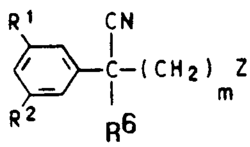

VII,

worin $R^1$, $R^2$, $R^6$ und m die oben angegebenen Bedeutungen haben und Z eine Austrittsgruppe darstellt, mit einem Phenylalkylamin der Formel VIII

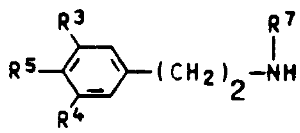

VIII,

worin $R^3$-$R^5$ und $R^7$ die obige Bedeutung besitzen, zur Reaktion bringt oder
e) Phenylacetonitrile der Formel IX

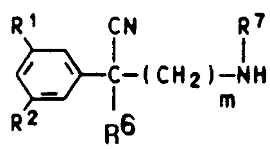

IX,

worin $R^1$, $R^2$, $R^6$, $R^7$ und m die beschriebene Bedeutung haben, mit Phenylalkyl-derivaten der Formel X

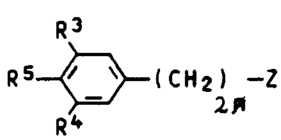

X,

worin $R^3$, $R^4$, $R^5$ und Z die oben angegebene Bedeutung haben, zur Reaktion bringt oder

f) basisch substituiere Phenylaceonitrile der Formel XI

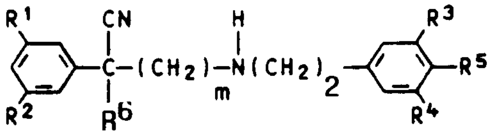

XI,

worin $R^1$-$R^6$ und m die angegebene Bedeutung haben, alkyliert oder
g) Phenylacetonitrile der Formel IX mit Aldehyden der Formel XII

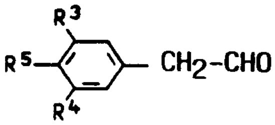

XII

worin $R^3$-$R^5$ dasselbe wie oben bedeuten, unter reduktiven Bedingungen umsetzt oder
h) Aldehyde der Formel XIII

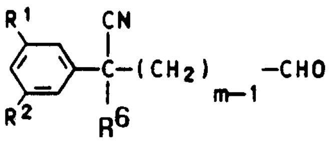

XIII

worin $R^1$, $R^2$, $R^6$ und m dasselbe wie oben bedeuten mit Phenylalkylaminen der Formel VIII unter reduktiven Bedingungen umsetzt,
i) Dinitrile der Formel XIV

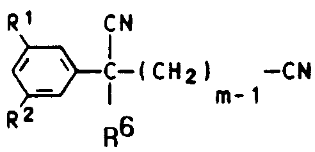

XIV

worin $R^1$, $R^2$ und m dasselbe wie oben bedeuten in Gegenwart eines Phenalkylamins der Formel VIII reduziert oder
k) Nitrile der Formel XV

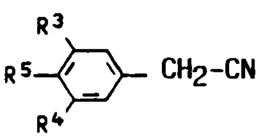

XV

worin $R^3$ bis $R^5$ dasselbe wie oben bedeuten in Gegenwart eines Phenylacetonitrils der Formel IX reduziert und die erhaltenen Verbindungen gewünschtenfalls in ihre Antipoden trennt und/oder in ein Salz mit einer physiologisch verträglichen Säure überführt.

## Claims

### Claims for the following Contracting States : BE, CH, DE, FR, GB, IT, LI, NL, SE

**1.** A phenylacetonitrile which is substituted by basic groups and is of the formula I

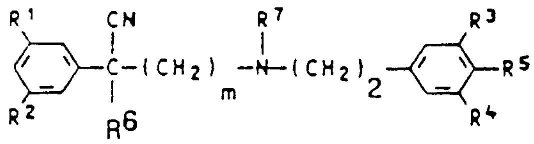

I

where $R^1$ and $R^2$ are identical or different and are each hydrogen, halogen, $C_1$-$C_3$-alkyl, methoxy or ethoxy,
$R^3$ and $R^4$ are identical or different and are each hydrogen, halogen, trifluoromethyl, $C_1$-$C_3$-alkyl, methoxy or ethoxy,
$R^5$ is hydrogen, halogen or $C_1$-$C_3$-alkyl,
although at least one of the radicals $R^1$ to $R^5$ must not be hydrogen, and if $R^6$ is isopropyl, $R^7$ is methyl, and, m is 3 then $R^1$, $R^2$, $R^3$ and $R^4$ cannot simultaneously be methoxy or ethoxy,
$R^6$ is a saturated hydrocarbon radical of not more than 6 carbon atoms or is phenyl,
$R^7$ is $C_1$-$C_4$-alkyl and
m is from 2 to 4,
and its antipodes and salts with physiologically tolerated acids.

**2.** A compound as claimed in claim 1 for use in the treatment of disorders.

### Claims for the following Contracting States : AT, ES

**1.** A process for the preparation of a phenylacetonitrile which is substituted by basic groups and is of the formula I

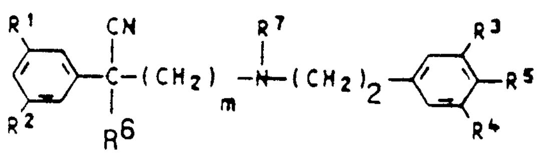

I

where $R^1$ and $R^2$ are identical or different and are each hydrogen, halogen, $C_1$-$C_3$-alkyl, methoxy or ethoxy,
$R^3$ and $R^4$ are identical or different and are each hydrogen, halogen, trifluoromethyl, $C_1$-$C_3$-alkyl, methoxy or ethoxy,
$R^5$ is hydrogen, halogen or $C_1$-$C_3$-alkyl,
although at least one of the radicals $R^1$ to $R^5$ must not be hydrogen, and if $R^6$ is isopropyl, $R^7$ is methyl, and, m is 3 then $R^1$, $R^2$, $R^3$ and $R^4$ cannot simultaneously be methoxy or ethoxy,
$R^6$ is a saturated hydrocarbon radical of not more than 6 carbon atoms or is phenyl,
$R^7$ is $C_1$-$C_4$-alkyl and
m is from 2 to 4,
or its antipodes or salts with physiologically tolerated acids, wherein

a) a phenylacetonitrile of the formula II

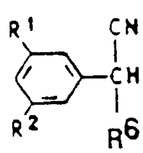

II

where $R^1$, $R^2$ and $R^6$ have the stated meanings, is reacted with a 1-phenylazaalkane of the formula

III

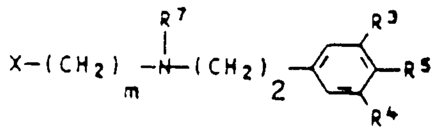

III

where $R^3$, $R^4$, $R^5$ and $R^7$ and m have the stated meanings and X is a leaving group, or
b) a phenylacetonitrile which is substituted by basic groups and is of the formula IV

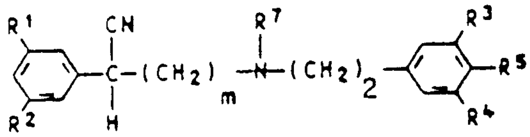

IV

where $R^1$, $R^2$, $R^3$, $R^4$, $R^5$ and $R^7$ and m have the stated meanings, is reacted with a compound of the formula V

$R^6$-Y V

where $R^6$ has the stated meanings and Y is a leaving group, or
c) a phenylacetonitrile of the formula VI

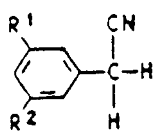

VI

where $R^1$ and $R^2$ have the stated meanings, is reacted with a 1-phenylazaalkane of the formula III and a compound of the formula V, or
d) a phenylacetonitrile of the formula VII

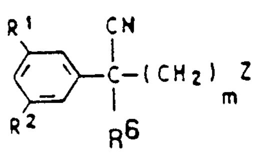

VII

where $R^1$, $R^2$, $R^6$ and m have the abovementioned meanings and Z is a leaving group, is reacted with a phenylalkylamine of the formula VIII

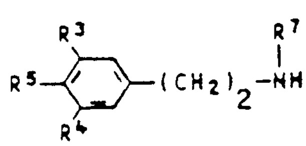

VIII

where $R^3$, $R^4$, $R^5$ and $R^7$ have the abovementioned meanings, or

e) a phenylacetonitrile of the formula IX

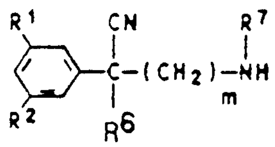

IX

where $R^1$, $R^2$, $R^6$, $R^7$ and m have the meanings described, is reacted with a phenylalkyl derivative of the formula X

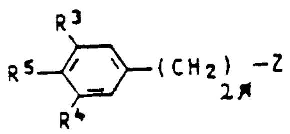

X

where $R^3$, $R^4$, $R^5$ and Z have the abovementioned meanings, or

f) a phenylacetonitrile which is substituted by basic groups and is of the formula XI

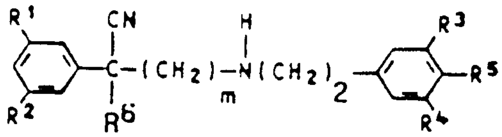

XI

where $R^1$, $R^2$, $R^3$, $R^4$, $R^5$, $R^6$ and m have the stated meanings, is alkylated or

g) a phenylacetonitrile of the formula IX is reacted with an aldehyde of the formula XII

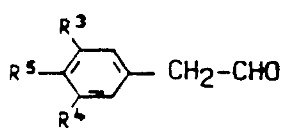

XII

where $R^3$, $R^4$ and $R^5$ have the same meanings as above, under reductive conditions or

h) an aldehyde of the formula XIII

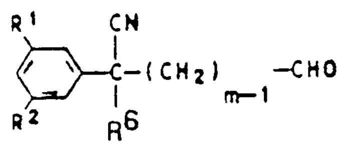

XIII

where $R^1$, $R^2$, $R^6$ and m have the same meanings as above, is reacted with a phenylalkylamine of the formula VIII under reductive conditions,

i) a dinitrile of the formula XIV

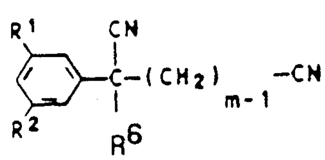

XIV

where $R^1$, $R^2$ and m have the same meanings as above, is reduced in the presence of a phenylalkylamine of the formula VIII or
k) a nitrile of the formula XV

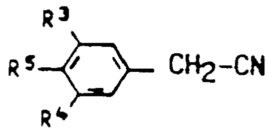

XV

where $R^3$, $R^4$ and $R^5$ have the same meanings as above, is reduced in the presence of a phenylacetonitrile of the formula IX and the resulting compound is, if desired, resolved into its antipodes or converted into a salt with a physiologically tolerated acid.

**Revendications**
**Revendications pour les Etats contractants suivants : BE, CH, DE, FR, GB, IT, LI, NL, SE**

1. Phénylacétonitriles à substituants basiques, répondant à la formule I

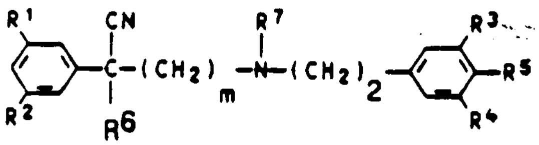

I,

dans laquelle $R^1$ et $R^2$, ayant des significations identiques ou différentes, représentent chacun un atome d'hydrogène, un atome d'halogène, un groupe alkyle en C1-C3, méthoxy ou éthoxy,
$R^3$, $R^4$, ayant des significations identiques ou différentes, représentent chacun un atome d'hydrogène, un atome d'halogène, un groupe trifluorométhyle, alkyle en C1-C3, méthoxy ou éthoxy,
$R^5$ représente un atome d'hydrogène ou d'halogène ou un groupe alkyle en C1-C3,
l'un au moins des symboles $R^1$ à $R^5$ devant avoir une signification autre que l'hydrogène et $R^1$ à $R^4$ ne pouvant représenter simultanément des groupes méthoxy ou éthoxy lorsque $R^6$ représente un groupe isopropyle, $R^7$ un groupe méthyle et m = 3,
$R^6$ représente un radical hydrocarboné saturé contenant jusqu'à 6 atomes de carbone ou un radical phényle,
$R^7$ représente un groupe alkyle en C1-C4, et
m est un nombre allant de 2 à 4,
ainsi que leurs antipodes et leurs sels d'acide acceptables pour l'usage pharmaceutique.

2. Composés selon la revendication 1, pour l'utilisation dans le traitement de maladies.

**Revendication pour les Etats contractants suivants : AT, ES**

1. Procédé de préparation de phénylacétonitriles à substituants basiques répondant à la formule I

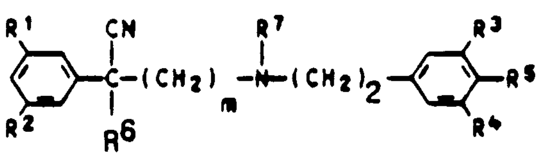

I,

dans laquelle $R^1$ et $R^2$, ayant des significations identiques ou différentes, représentent chacun un atome d'hydrogène, un atome d'halogène, un groupe alkyle en C1-C3, méthoxy ou éthoxy,
$R^3$, $R^4$, ayant des significations identiques ou différentes, représentent chacun un atome d'hydrogène, un atome d'halogène, un groupe trifluorométhyle, alkyle en C1-C3, méthoxy ou éthoxy,
$R^5$ représente un atome d'hydrogène, un atome d'halogène ou un groupe alkyle en C1-C3,

l'un au moins des symboles $R^1$ à $R^5$ devant avoir une signification autre que l'hydrogène, et $R^1$ à $R^4$ ne pouvant représenter simultanément des groupes méthoxy ou éthoxy lorsque $R^6$ représente un groupe isopropyle, $R^7$ un groupe méthyle et m = 3,
$R^6$ représente un radical hydrocarboné saturé contenant jusqu'à 6 atomes de carbone ou un radical phényle,
$R^7$ représente un groupe alkyle en C1-C4, et
m est un nombre allant de 2 à 4,
et de leurs antipodes et sels d'acides acceptables pour l'usage pharmaceutique, caractérisé en ce que :

a) on fait réagir des phénylacétonitriles de formule II

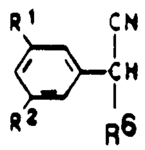

II,

dans laquelle $R^1$, $R^2$ et $R^6$ ont les significations indiquées ci-dessus, avec des 1-phényl-aza-alcanes de formule III

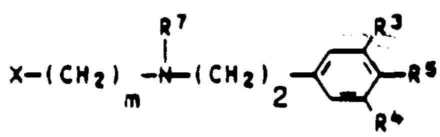

III,

dans laquelle $R^3$ à $R^5$, $R^7$ et m ont les significations ci-dessus, et
X représente un groupe éliminable, ou bien
b) on fait réagir des phénylacétonitriles à substituants basiques de formule IV

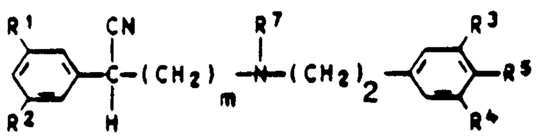

IV,

dans laquelle $R^1$ à $R^5$, $R^7$ et m ont les significations indiquées ci-dessus, avec des composés de formule V

$R^6$-Y V

dans laquelle $R^6$ a les significations indiquées ci-dessus et Y représente un groupe éliminable, ou bien
c) on fait réagir des phénylacétonitriles de formule VI

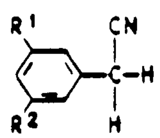

VI,

dans laquelle $R^1$ et $R^2$ ont les significations indiquées ci-dessus, avec des 1-phényl-aza-alcanes de formule III et des composés de formule V, ou bien

d) on fait réagir des phénylacétonitriles de formule VII

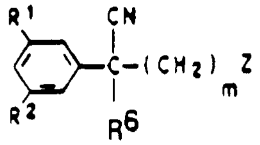

VII.

dans laquelle $R^1$, $R^2$, $R^6$ et m ont les significations indiquées ci-dessus, et Z représente un groupe éliminable, avec une phénylalkylamine de formule VIII

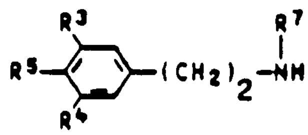

VIII,

dans laquelle $R^3$ à $R^5$ et $R^7$ ont les significations indiquées ci-dessus, ou bien
e) on fait réagir des phénylacétonitriles de formule IX

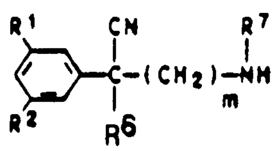

IX,

dans laquelle $R^1$, $R^2$, $R^6$, $R^7$ et m ont les significations indiquées ci-dessus, avec des dérivés phénylalkylés de formule X

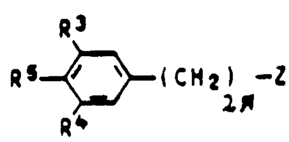

X,

dans laquelle $R^3$, $R^4$, $R^5$, et Z ont les significations indiquées ci-dessus, ou bien
f) on alkyle des phénylacétonitriles à substituants basiques de formule XI

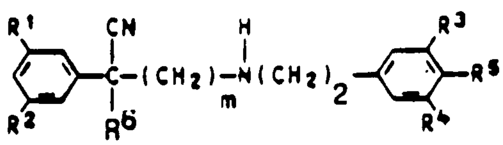

XI,

dans laquelle $R^1$ à $R^6$ et m ont les significations indiquées ci-dessus, ou bien
g) on fait réagir dans des conditions réductrices des phénylacétonitriles de formule IX avec des aldéhydes de formule XII

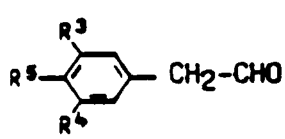

XII

dans laquelle $R^3$ à $R^5$ ont les significations indiquées ci-dessus, ou bien

h) on fait réagir dans des conditions réductrices des aldéhydes de formule XIII

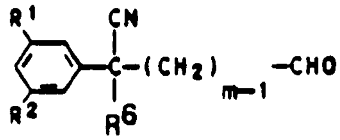

XIII

dans laquelle $R^1$, $R^2$, $R^6$ et m ont les significations indiquées ci-dessus, avec des phénylalkylamines de formule VIII,

i) on réduit des dinitriles de formule XIV

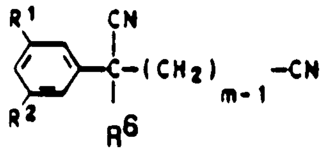

XIV

dans laquelle $R^1$, $R^2$ et m ont les significations indiquées ci-dessus, en présence d'une phénylalkylamine de formule VIII, ou bien

k) on réduit des nitriles de formule XV

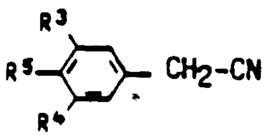

XV

dans laquelle $R^3$ à $R^5$ ont les significations indiquées ci-dessus, en présence d'un phénylacétonitrile de formule IX, et, si on le désire, on sépare les composés obtenus en leurs antipodes et/ou on convertit les composés obtenus en sel d'acides acceptables pour l'usage pharmaceutique.